# EUROPEAN PATENT APPLICATION

(11) **EP 3 569 260 A1**
(43) Date of publication of application: **20.11.2019**
(21) Application number: 19175272.4
(22) Date of filing: 17.05.2019
(51) Int. Cl.: A61L 15/26, A61L 15/42, A61L 15/46, A61L 15/64

(54) **NON-WOVEN FABRIC BANDAGE AND A METHOD FOR THE PRODUCTION OF A NON-WOVEN FABRIC BANDAGE**

(30) Priority: 18.05.2018 PL 42563618
(71) Applicant: Centrum Materialow Polimerowych I Weglowych Polski Akademii Nauk, 41-819 Zabrze (PL); Slaski Uniwersytet Medyczny w Katowicach, 40-055 Katowice (PL)
(72) Inventor: DOBRZY SKI, Piotr, 41-808 Zabrze (PL); KASPERCZYK, Janusz, 40-693 Katowice (PL); SOBOTA, Micha, 42-224 Cz stochowa (PL); STOJKO, Mateusz, 43-170 aziska Górne (PL); W ODARCZYK, Jakub, 34-520 Poronin (PL); KOMOSI SKA-VASSEV, Katarzyna, 42-622 Nowe Chech o (PL); OLCZYK, Pawe, 42-500 B dzin (PL); STOJKO, Jerzy, 43-170 aziska Górne (PL); WALUGA, Ewa, 41-200 Sosnowiec (PL)
(74) Representative: Belz, Anna

(57) **Abstract**

A non-woven dressing fabric intended specifically for the treatment of non-healing wounds composed of randomly arranged fibers of a biodegradable polymer, containing propolis, characterized in that the fibers are in the form of a composite with a matrix of aliphatic biodegradable copolymer, constituting 25 to 99 % by weight of the composite, whereas propolis particles are uniformly dispersed in the biodegradable copolymer matrix in an amount ranging from 1 to 75 % by weight, with non-woven fabric thickness ranges from 20 to 2000 µm, and an average diameter of the composite fibers ranges from 250 to 5000 nm. The subject of the invention is also a method of producing a non-woven dressing fabric produced by electrospinning method, based on formation of composite fibers from an aliphatic biodegradable copolymer solution in a concentration of 1 to 20 % by weight and containing propolis in amount from 1 to 75 % by weight. The solution is placed in a dispenser with an infusion pump, terminated with a nozzle, and then the composite fibers are formed at a rate of dosage of the base solution from 0.1 to 15 ml/h and deposited on the surface of the collector, preferably cylindrical and rotating. The distance between the nozzle and the collector is from 5 cm to 30 cm, and the potential difference between the nozzle and the collector is from 5 to 50 kV.

## Description

The present invention relates to a non-woven fabric made of biodegradable polymer containing propolis and especially intended for the treatment of non-healing wounds and a method for producing a non-woven fabric.

The selection of the optimal wound dressing is crucial considering the wound healing process. Traditional dressings, including gauze, adhesive plaster, bandages, are used to protect the wound against contamination and protect it mechanically. However, modern dressings are designed so that their role is not only to cover the surface of the wound, but also to participate with the complex healing process, accelerate it and minimize the risk of the complications.

As described in, among others, in the article of J. Dissemond et al.: "Modern wound care - practical aspects of non-interventional topical treatment of patients with chronic wounds" J Dtsch Dermatol Ges. 2014 Jul, 12 (7) : 541-54, a modern dressing should humidify the wound environment, accelerate the formation of epidermis, accelerate angiogenesis and synthesis of connective tissue, allow gas exchange between the wound and the environment, provide the optimal wound temperature to increase blood flow within it, separate it from the source of infection, not to adhere to the wound, minimize its unpleasant smell, support the migration of leukocytes and enzymes, be transparent, allow to observe the healing process, be sterile, nontoxic and non-allergic.

Article of G.D. Mogosanu et al.: "Natural and synthetic polymers for wounds and burns dressing", Int J Pharm. 2014 Mar 25, 463 (2) : 127-36, describes the dressings containing natural polymers, which are in general perfectly tolerated by the skin. These polymers include, among others, polysaccharides: alginates, chitin, chitosan, heparin or chondroitin as well as proteoglycans and proteins: collagen, gelatin, fibrin, keratin, non-woven silk, egg shell membrane.

An interesting alternative to simple dressings made of natural polymers are composite dressings containing a polymer with an incorporated active ingredient, as described, for example, in S. Chew et al.: "The Role of Electrospinning in the Emerging Field of Nanomedicine", Curr Pharm Des. 2006, 12 (36): 4751-4770. Using complex dressings, not only the optimal wound healing parameters due to the presence of the active ingredient can be obtained, but also, due to the application of advanced manufacturing systems such as electrospinning, the pharmacokinetic processes can be controlled to achieve a prolonged action of the active compound within the healing wound. Moreover, as described e.g. in the publication of M. Arenbergerova et al.: "Light-activated nanofibre textiles exert antibacterial effects in the setting of chronic wound healing", Exp Dermatol., 2012 Aug, 21 (8): 619-24, using antibacterial ingredients such as nanosilver, antibiotics, antioxidants and bee products, a sterility of wound environment can be achieved which is one of the biggest challenges of the medicine concerning the modern dressings.

Patent KR100791039 B1 describes the nanofiber wound dressing made by electrospinning from a solution of a biocompatible synthetic or natural polymer such as polyvinyl alcohol, poly(ethylene oxide), polyethylene glycol, polylactide, polyglycolide, collagen, gelatin, alginate, alginic acid, chitosan etc., containing an antioxidant in the form of N-acetyl-L-cysteine (NAC) in an amount of 3-20 g / dl.

Patent US2015290354 A1 describes a multi-layer bandage for use as a wound dressing or as a targeted drug delivery device. The bandage contains nanofiber produced by electrospinning from a solution of biocompatible polymers with a concentration of 5 to 25% by weight, prepared from polymers such as, e.g. polylactide, polyvinyl alcohol, polycaprolactone, poly(ethylene oxide), polyglycolide, poly(2-hydroxyethyl methacrylate), poly(methyl methacrylate), dissolved in e.g. dichloromethane, dichloroethyl, dimethylformamide, tetrahydrofuran or acetone. The produced fibers are porous or hollow, and a therapeutic agent, anti-inflammatory drugs, growth factors, cells, etc. are placed on their porous surface or inside the fibers. Silver nanoparticles with an average diameter of 1- 10 µm are often used as anti-inflammatory components.

The preparation of polymeric fibers by electrospinning consists in forming the fibers from molten or dissolved polymers in an electric field generated between two electrodes. One of the electrodes is usually a nozzle located at the end of dispenser containing a molten polymer or polymer solution, connected with an infusion pump. The nozzle is connected to a high voltage generator. The second electrode is a grounded, aluminum electrode, called a collector, on the surface of which the formed fibers are deposited. The number of research works growing every year confirms the continuous interest in this process. In an articles of H. Xiuli et al.: "Electrospinning of polymeric nanofibers for drug delivery applications", J. of Controlled Release, 185 (2014) 12 - 21 and M. Kwiatkowska et al., "Formation of poly(lactic acid) fibers by electrospinning method from solution in various solvent systems ", Polimery 2015, 60, no. 7 - 8, pp. 480 - 485, authors described that it is possible to produce fibers of a specific structure by means of appropriate control of the electrospinning process parameters, which is complicated due to the parameters number. They include e.g. values related to the properties of solution (molar mass of the polymer, type of solvent, solution viscosity), apparatus (solution delivery rate, the applied voltage, nozzle diameter and it distance from the collector) and the external environment (temperature, humidity). Usually, experimental methods as well as mathematical modelling are used to optimize the electrospinning process.

Patent description PL197202 B1 discloses a method for the production of nanofibers, consisting in preparing a polymer spinning solution, applying this solution by a capillary nozzle, to an electrostatic field that is generated between an electrode connected to the current generator and a grounded collector. The fibers formed in electrostatic field are collected on the surface of the latter. The spinning solution is prepared from natural or synthetic polymers dissolved in water, organic solvents or in aqueous solutions of these solvents, preferably from cellulose dissolved in N-methylmorpholine N-oxide or from poly(ethylene oxide) dissolved in water or alcohol. Optionally, modifying substances with a particle size less than 30 nm are added to the solution and before the introduction of the spinning solution into the electrostatic field, an electric current is supplied to it using an electrode connected to the power source. Moreover, the shape of both electrodes are selected depending on the required shape and geometrical shape of the fibers to be formed.

Bee products are nowadays one of the most intensively studied active ingredients of modern dressings. For example, bee honey provides a sterile wound healing environment, as described in L. Vandamme et al.: "Honey in modern wound care: a systematic review. Burns ", Dec 2013, 39 (8): 1514-25. Wound exudate increases the production of hydrogen peroxide by glucose oxidase, which activates neutrophils and enhances the body's inflammatory response. In contrast, flavonoids present in honey inhibit the production of peroxide free radicals, thus protecting tissues from excessive damage resulting from induced inflammation process.

Patent PL / EP2310056 T3 described the wound dressing in the form of a flexible sheet with a thickness of 0.25 to 10 mm, containing polymer fibers in a form honey-impregnated network structure. The fibers are produced from a terpolymer of acrylic acid, methyl acrylate and a small amount of acrylate/methacrylate monomer, with acrylic acid partially neutralized to the sodium salt of acrylic acid. The flexible sheet further comprises at least one layer of compressed cellulose material (e.g., tissue-paper) or polyester, interleaved with at least one layer of polymer fiber.

In turn, in the publication of E. Adomaviciute et al.: "Formation and Biopharmaceutical Characterization of Electrospun PVP Mats with Propolis and Silver Nanoparticles for Fast Releasing Wound Dressing", BioMed Research International Volume 2016, Article ID 4648287, it was shown that the use of propolis in electrospinning-prepared polymeric dressings, made of e.g. polyvinylpyrrolidone, polylactide or polyurethane, allows to obtain a dressing material with excellent antibacterial and reparative-regenerative properties. Propolis (bee kit), as described e.g. in the publication of V.D. Wagh: "Propolis: A Wonder Bees Product and Its Pharmacological Potentials", Advances in Pharmacological Sciences, 2013, Vol. 2013, Article ID 308249, is a natural resinous-wax substance produced by honeybees as a repair and protective material. It is a mixture of resins, juices, gums, polysaccharides, oils and bee exudates formed as an indigestible fraction of pollen. Due to its waxy nature and mechanical properties, propolis is used by bees in the construction and repair of beehives, as well as for sealing holes and cracks and smoothing of the inner walls of hives and also as a protective barrier against pests and adverse weather conditions. This substance exhibits a proven antioxidant, anti-inflammatory, immunomodulatory, antiviral, anticancer, antibacterial and antifungal activity, stimulates re-epithelialization and shortens wound healing time. The fact that propolis exerts an extremely beneficial effect on human health is described, among others, in A.Kuropatnicki et al., "Historical Aspects of Propolis Research in Modern Times", Evidence-Based Complementary and Alternative Medicine; 2013; Vol. 2013, Article ID 964149. Propolis was already used in ancient times in folk medicine, in the treatment of many diseases and embalming of corpses (Egypt). The Incas used propolis as an anti-fever medication, while the ancient Greeks and Romans used it to disinfect the mouth and treat hard-to-heal wounds. From the 17th century, the use of propolis in medicine and pharmacy was increasing, while during the Second World War in the then Soviet Union propolis was used to treat tuberculosis, weakness and lack of appetite. In the publication of V.D. Wagh: "Propolis: A Wonder Bees Product and Its Pharmacological Potentials. Advances in Pharmacological Sciences ", 2013; Vol. 2013; Article ID 308249 (https://www.hindawi.com/26393196) it was shown that propolis is currently used as an ingredient of biopharmaceuticals and becomes popular as a natural preservative and source of bioactive compounds in food and beverages, which helps to extend the half-life of food products. In medicine, propolis is used as an adjuvant in the treatment of colds, upper respiratory tract infections, and viral infections. In cosmetology, it is used as an ingredient in skin care products. In dermatology it is used in the treatment of acne, herpes, preventive treatment of caries, gingivitis and oral cavity, as well as in preparations used in wound healing and burns.

Despite the wide range of wound dressings available on the market, in the number of over 3000 types, as was reported by S. Dhivya et al., in the article: "Wound dressings - a review", Biomedicine (Taipei), 2015 Dec, 5 (4): 22, it is impossible to distinguish the dressing that would be universal and effective in a wide range of chronic wound therapy. Therefore, new solutions are still required to not only accelerate the healing process, but at the same time do not require to change the dressings, which could cause permanent damage to the wound and interfere with the healing process.

The aim of the invention is to develop a non-woven fabric from a biodegradable polymer, containing propolis, especially intended for the treatment of non-healing wounds, which releases propolis in a controlled manner throughout the entire treatment period and creates favorable conditions for the regeneration of damaged cells while ensuring a sterile wound environment, and after the end of treatment is being degraded, which allows to avoid discomfort associated with the removal of the dressing.

Non-woven fabric consisting of randomly arranged fibers made of a biodegradable polymer containing propolis, according to the invention, contains the fibers that are in the form of a composite with a biodegradable aliphatic copolymer matrix, constituting 25 to 99% by weight of the composite and selected from the group consisting of:
(a) poly(lactide-co-glycolide) with lactydyl content of 50 to 95 mole %, glycolidyl content of 5 to 50 mole %,
(b) poly(lactide-co-ε-caprolactone) with lactydyl content of 5 to 95 mole %, ε-caproil content of 5 and 95 mole %.
(c) poly(glycolide-co-ε-caprolactone) with glycolidyl content of 10 to 50 mole %, ε-caproil content of 50 and 90 mole %,
(d) poly(lactide-co-glycolide-co-ε-caprolactone) with lactydyl content of 15 to 85 mole %, glycolidyl content of 5 to 50 mole %, ε-caproil content from 5 to 30 mole %,
(e) poly(lactide-co-trimethylene carbonate) with a lactydyl content of 5 to 95 mole %, trimethylene carbonate content from 5 to 95 mole %,
(f) poly(glycolide-co-trimethylene carbonate) with a glycolidyl content of 5 to 30 mole %, trimethylene carbonate content of 70 to 95 mole %,
(g) poly(lactide-co-glycolide-co-trimethylene carbonate) with a lactydyl content of 15 to 85 mole %, glycolidyl content of 5 to 50 mole %, trimethylene carbonate content from 5 to 50 mole %.

Propolis particles are uniformly dispersed in the biodegradable copolymer matrix in an amount of 1 to 75 % by weight, with the non-woven fabric thickness being in the range from 20 to 2000 µm, and the average diameter of the composite fibers forming it is from 250 to 5000 nm.

Preferably, the propolis is dispersed in the matrix of a biodegradable polymer in an amount of 5 to 20 % by weight.

Preferably, the thickness of the non-woven fabric is from 200 to 500 µm, and the average diameter of the composite fibers forming the non-woven nanofibric is from 500 to 1000 nm.

The essence of the method of producing a non-woven dressing fabric, composed of randomly arranged fibers of a biodegradable polymer, containing propolis, made by electrospinning method, which consists in formation in the electric field fibers from a polymer solution which is placed in a nozzle-equipped dispenser with an infusion pump, and then deposition of the fibers randomly on the collector surface, is characterized, according to the invention, by the fact that the fibers are in the form of a composite with a matrix of an aliphatic biodegradable copolymer, which constitutes 25 to 99 % by weight of the composite and is selected from the group consisting of:
(a) poly(lactide-co-glycolide) with a lactydyl content of 50 to 95 mole %, glycolidyl content of 5 to 50 mole %,
(b) poly(lactide-co-ε-caprolactone) with a lactydyl content of 5 to 95 mole %, ε-caproil content of 5 and 95 mole %.
(c) poly(glycolide-co-ε-caprolactone) with a glycolidyl content of 10 to 50 mole %, ε-caproil content of 50 and 90 mole %,
(d) poly(lactide-co-glycolide-co-ε-caprolactone) with a lactydyl content of 15 to 85 mole %, glycolidyl content of 5 to 50 mole %, ε-caproil content from 5 to 30 mole %,
(e) poly(lactide-co-trimethylene carbonate) with a lactydyl content of 5 to 95 mole %, trimethylene carbonate content from 5 to 95 mole %,
(f) poly(glycolide-co-trimethylene carbonate) with a glycolidyl content of 5 to 30 mole %, trimethylene carbonate content of 70 to 95 mole %,
(g) poly(lactide-co-glycolide-co-trimethylene carbonate) with a lactydyl content of 15 to 85 mole %, glycolidyl content of 5 to 50 mole %, trimethylene carbonate content from 5 to 50 mole %.
wherein the aliphatic biodegradable copolymer is dissolved in a fluorinated alcohol, preferably 1,1,1,3,3,3-hexafluoro-2-propanol, at room temperature, obtaining the solution of a concentration from 1 to 20 % by weight, followed by addition of propolis in amount from 1 to 75 % by weight to above solution and mixing for up to 24 hours until it is completely dissolved. The obtained base solution is introduced into a dispenser with infusion pump terminated with a spinning nozzle placed on a movable arm and moving along the collector, preferably cylindrical and rotating, and the nozzle distance from the collector is from 5 cm to 30 cm, and the potential difference between the nozzle and the collector is from 5 to 50 kV. Composite fibers with a mean diameter of 250 to 5000 nm are then extracted, at a feed rate of the base solution from 0.1 to 15 ml/h, preferably from 1 to 2.5 ml/h, subjected to winding on the surface of the collector until a non-woven fabric is obtained with a thickness in the range from 20 to 2000 µm, and after the electrospinning process is finished, the non-woven fabric is dried in a vacuum dryer for up to 10 days and then subjected to e-beam or gamma irradiation sterilization.

Preferably the propolis is added in an amount of from 5 to 20 % by weight.

It is also advantageous if the composite fibers synthesized during the process have an average diameter of 500 to 1000 nm and are arranged to obtain a non-woven fabric with a thickness in the range of 200 to 500 µm.

Preferably, a biodegradable copolymer solution is prepared at a concentration of 6 to 10 % by weight.

The non-woven fabric dressing according to the invention, made of a composite with a matrix of a biodegradable polymer and evenly dispersed propolis as a medicinal substance, can be successfully used for the treatment of wounds, especially those difficult to heal. After application, there is no need to change the dressing throughout the treatment period, since propolis released from the biodegradable polymer matrix continuously for a period of at least 2 months provides sterile wound environment. In that it is possible to avoid continuous irritation of the wound and disturbance of the healing process. This form of propolis application provides its controlled and continuous release, more systematically when compared with propolis administered by the other transdermal routes, such as ointments and creams. In addition, the discomfort associated with the removal of the dressing is avoided because it is completely degraded over a given period of time.

The use of the electrospinning process the synthesis of the fibers from the polymer solution enables the uniform distribution of propolis in the whole mass (volume) of a single fiber. This enables the sustained release of therapeutic substance into the wound area during the degradation of the non-woven dressing fabric. Moreover, the electrospinning process allows to control the diameters of the obtained fibers, and thus, the development of the specific surface of the non-woven fabric. Therefore, one can control the propolis release rate and thus select its appropriate therapeutic dose for a specific medical case.

It is also important to choose the appropriate type of polymer from the group of aliphatic copolyesters or copolyestercarbonates which determines the time of fiber degradation and the kinetics of propolis release. The choice of polymer type depends on the expected degradation time of polymeric fibers, and the degradation time results from the molecular structure of copolymer chains, which directly affects the degradation time of the fibers made of them and thus allows the control the time of absorption (resorption) of the non-woven dressing to meet the therapeutic requirements, e.g. allows to obtain adequately sustained release of the active substance required in the treatment of non-healing wounds. However, the molecular structure of copolymer chains corresponds to the content of individual comonomeric units in chains (their composition). The copolymers containing lactide, glycolide and ε-caprolactone, release propolis by bulk biodegradation of polymeric carrier, while the use of polymers containing trimethylene carbonate additionally provides the release control by biodegradation of the surface.

Due to the possibility of controlling the morphology and composition, non-woven dressing fabrics with propolis obtained by electrospinning method from the solution, can be potentially used in personalized therapy.

The copolymers used to form the non-woven fabric, apart from providing the full biodegradability and biocompatibility of the dressing, also ensure its high elasticity and strength, so there is no risk of its undesired disintegration during treatment. The non-woven fabric according to the invention is easy to apply. It is enough to place it on the wound and fix it using dressing gauze or ready-to-use transparent dressings. In case of any side effects, the non-woven fabric can be easily removed from the wound surface. The non-woven fabric may also constitute one of the layers of ready-made dressings, that placed on the wound side.

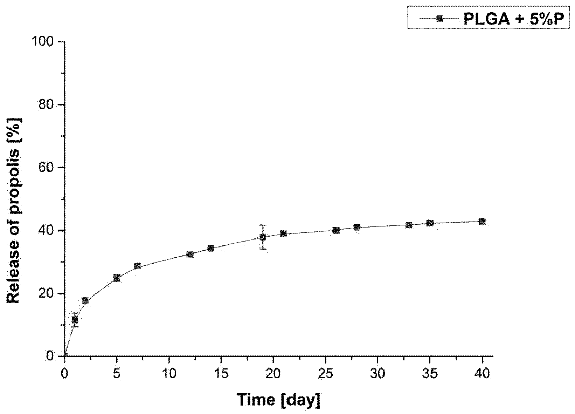

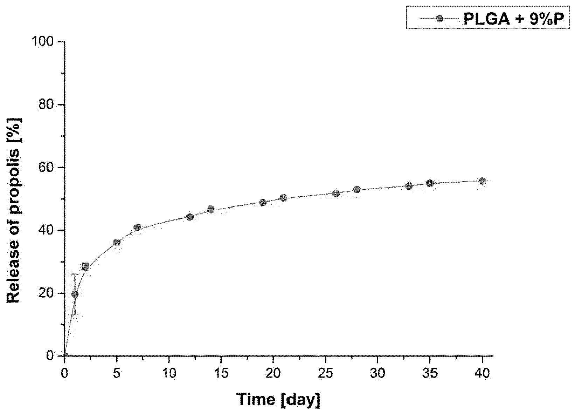

A non-woven fabric dressing and a method for producing a non-woven fabric dressing according to the invention are explained below in practical embodiments and in figures that illustrates the results of *in vitro* release of propolis from a non-woven fabric made of a composite containing 95 % by weight of poly(D,L-lactide-co-glycolide) and 5 % by weight of propolis (fig. 1) and of a composite comprising 91 % by weight of poly(D,L-lactide-co-glycolide) and 9 % by weight of propolis (fig. 2).

### Example 1

The non-woven fabric dressing consists of randomly oriented composite fibers, composed of 95 % by weight of a biodegradable aliphatic copolymer, poly(D,L-lactide-co-glycolide) with the structural formula shown below, containing 83 mole % lactydyl and 17 mole % glycolidyl units.

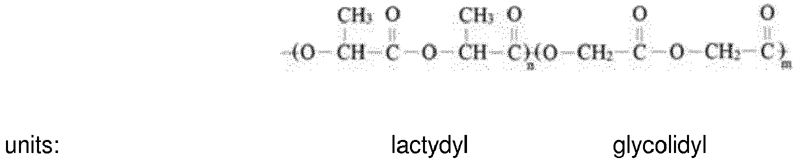

In the fiber matrix, particles of propolis are uniformly dispersed in an amount of 5 % by weight. The thickness of the non-woven fabric is 350 µm, and the average diameter of the composite fibers is 1000 nm.

### Example 2

For the fabrication of non-woven fabric dressing described in Example 1, 2.55 g of poly(D,L-lactide-co-glycolide) with a lactydyl content of 83 mole % and glycolidyl content of 17 mole % was dissolved in 25 ml of 1,1,1,3,3,3-hexafluoro-2-propanol at room temperature to obtain a 6 % by weight solution, and then 0.13 g propolis was added and the mixture was stirred on a magnetic stirrer at room temperature for 24 hours until it was completely dissolved. The solution obtained was introduced into a dispenser with a pump terminated with a nozzle, placed on a movable arm and moving along a cylindrical collector. The nozzle distance from the collector was 15 cm, and the voltage applied was 21 kV nozzle. The cylinder with a diameter of 27 mm constituting the collector rotated at 800 rpm and was connected to a power supply with a negative potential of -6 kV. Composite fibers with an average diameter of 1000 nm were synthesized at a solution feed rate of 1.5 ml/h and randomly deposited on the collector surface until a 350 µm non-woven fabric layer was obtained. The total volume of the solution used was 22 ml. The non-woven fabric was dried in a vacuum dryer at a pressure of 10 mbar at 25°C for 10 days and then sterilized in electron beam at a dose of 10 kGy.

The obtained wound dressing was subjected to propolis release tests *in vitro* at 37°C in PBS (*phosphate-buffered saline,* pH 7.4). Using high-performance liquid chromatography, daily doses of propolis released into the buffer were measured and visualized in the graph (fig. 1).

### Example 3

The non-woven fabric dressing consists of randomly arranged composite fibers, composed of 91 % by weight of an aliphatic biodegradable copolymer, poly(D,L-lactide-co-glycolide) containing 83 mole % of lactydyl units and 17 mole % glycolidyl units. In the matrix, particles of propolis are uniformly dispersed in an amount of 9 % by weight. The thickness of the non-woven fabric is 330 µm, and the average diameter of the composite fibers is 1000 nm.

### Example 4

For the fabrication of non-woven fabric dressing described in Example 3, 2.55 g of poly(D,L-lactide-co-glycolide) with a lactydyl content of 83 mole % and glycolidyl content of 17 mole % was dissolved in 25 ml of 1,1,1,3,3,3-hexafluoro-2-propanol at room temperature to obtain a solution of 6 % by weight, and then 0.26 g of propolis was added and stirred on a magnetic stirrer at room temperature for 24 hours until it was completely dissolved. The obtained solution was introduced into a dispenser with a pump terminated with a nozzle, placed on a movable arm and moving along a cylindrical collector. The nozzle distance from the collector was 15 cm, and the voltage applied to the nozzle 21 kV. The cylinder with a diameter of 27 mm constituting the collector rotated at 800 rpm and was connected to a power supply with a negative potential of -6 kV. Composite fibers of an average diameter of 1000 nm were synthesized at a solution feed rate of 1.5 ml/h and deposited randomly on the collector surface for 14.5 hours until a 330 µm thick non-woven fabric layer was obtained. The total volume of the solution used was 22 ml. The nonwoven fabric was dried in a vacuum dryer at 10 mbar at 25°C for 10 days and then sterilized by electron beam at a dose of 10 kGy.

The obtained non-woven fabric dressing was subjected to *in vitro* tests of propolis release at 37°C in PBS (*phosphate-buffered saline;* pH 7.4). Using high-performance liquid chromatography, doses of propolis daily released to the buffer were measured and illustrated in the graph (fig. 2).

### Example 5

The non-woven fabric dressing consists of randomly arranged composite fibers, composed of 83 % by weight of a biodegradable aliphatic copolymer, poly(D,L-lactide-co-glycolide) containing 70 mole % lactydyl and 30 mole % glycolidyl units. Propolis particles are uniformly dispersed in the matrix in an amount of 17 % by weight. The thickness of the non-woven fabric is 300 µm, with the average diameter of composite fibers of 1000 nm.

### Example 6

For the non-woven fabric dressing described in Example 5, 5.1 g of poly(D,L-lactide-co-glycolide) with a molar content of 70 mole % of lactydyl units and 30 mole % of glycolidyl units was dissolved in 50 ml of 1,1,1,3,3,3-hexafluoro-2-propanol at room temperature to obtain a solution of 6 % by weight, and then 1.02 g of propolis was added and stirred on a magnetic stirrer at room temperature for 24 hours until it was completely dissolved. The solution obtained was introduced into a dispenser with a pump terminated with a nozzle, placed on a movable arm and moving along a cylindrical collector. The nozzle distance from the collector was 16 cm, and the voltage applied to nozzle of the 22 kV. The cylinder with a diameter of 27 mm constituting the collector rotated at 800 rpm and was connected to a power supply with a negative potential of -7 kV. Composite fibers with an average diameter of 1000 nm were synthesized at a solution delivery rate of 2 ml/h and deposited randomly on the collector surface for 15 hours until a 400 µm thick non-woven fabric layer was obtained. The total volume of the solution used was 30 ml. The non-woven fabric was dried in a vacuum dryer at 10 mbar at 25°C for 10 days and then sterilized by electron beam at a dose of 10 kGy.

### Example 7

The non-woven fabric consists of randomly oriented composite fibers, composed of 87 % by weight of an aliphatic biodegradable terpolymer, poly(L-lactide-co-glycolide-co-trimethylene carbonate) with the structural formula shown below, containing 55 mole % of lactydyl units, 20 mole % of glycolidyl units and 25 mole % of trimethylene carbonate.

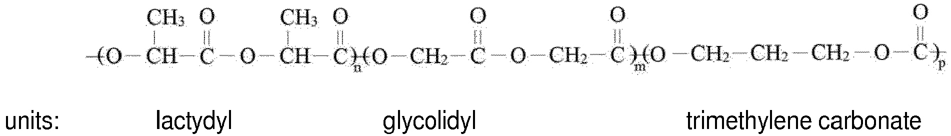

Within the matrix, particles of propolis are uniformly dispersed in an amount of 13 % by weight. The thickness of the non-woven fabric is 350 µm, with average diameter of composite fibers of 1000 nm.

### Example 8

For preparing the non-woven fabric described in Example 7, 4 g of poly(lactide-co-glycolide-co-trimethylene carbonate) containing 55 mole % lactydyl units, 20 mole % glycolidyl units and 25 mole % trimethyl carbonate units was dissolved in 40 ml 1,1,1,3,3,3-hexafluoro-2-propanol at room temperature to obtain a 6 % by weight solution, followed by the addition of 0.6 g propolis and the mixture was stirred on a magnetic stirrer at room temperature for 24 hours until its complete dissolution. The solution obtained was introduced into a dispenser with a pump terminated with a nozzle, placed on a movable arm and moving along a cylindrical collector. The distance of the nozzle from the collector was 17 cm, and the voltage applied to the nozzle 25 kV. The roller with a diameter of 27 mm constituting the collector rotated at 800 rpm and was connected to a power supply with a negative potential of -5 kV. Composite fibers with an average diameter of 1000 nm were synthesized at a solution feed rate of 2.5 ml/h and deposited randomly on the collector surface for 14 h until a non-woven fabric layer with a thickness of 370 µm was obtained. The total volume of the solution used was 35 ml. The non-woven fabric was dried in a vacuum dryer at 10 mbar at 25°C for 10 days and then sterilized by electron beam at a dose of 10 kGy.

### Example 9

The non-woven fabric consists of randomly oriented composite fibers, the matrix of which is 85 % by weight of the aliphatic biodegradable terpolymer, poly(L-lactide-co-glycolide-co-ε-caprolactone) with the structural formula shown below, containing 70 mole % lactydyl units, 13 mole % glycolidyl units and 17 mole % ε-caproil units.

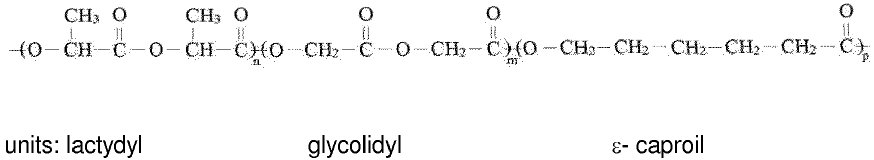

Propolis particles in the amount of 15 % by weight are uniformly dispersed within the matrix. The thickness of the non-woven fabric is 300 µm, and the average diameter of the composite fibers is 1000 nm.

### Example 10

For preparing the non-woven fabric described in Example 9, 75 g of poly(L-lactide-co-glycolide-co-ε-caprolactone) containing 70 mole % lactydyl units, 13 mole % glycolidyl units and 17 mole % ε-caproil units were dissolved in 50 ml 1,1,1,3,3,3-hexafluoro-2-propanol at room temperature, obtaining a solution of 8.5 % by weight, and then 1.02 g of propolis was added and stirred on a magnetic stirrer at room temperature for 24 hours until it is completely dissolved. The solution obtained was introduced into a dispenser with a pump terminated with a nozzle, placed on a movable arm and moving along a cylindrical collector. The nozzle distance from the collector was 17 cm, and the voltage applied to the nozzle 29 kV. The cylinder with a diameter of 27 mm constituting the collector rotated at 800 rpm and was connected to a power supply with a negative potential of -10 kV. Composite fibers with an average diameter of 1000 nm were synthesized at a solution feed rate of 2.5 ml/h and deposited randomly on the collector surface for 16 h until a non-woven fabric layer with a thickness of 360 µm was obtained. The total volume of the solution used was 40 ml. The non-woven fabric was dried in a vacuum dryer at 10 mbar at 25°C for 10 days and then sterilized by electron beam at a dose of 10 kGy.

### Example 11

The non-woven fabric consists of randomly oriented composite fibers, composed in 87 % by weight of a biodegradable aliphatic copolymer, poly(L-lactide-co-ε-caprolactone) containing 50 mole % lactydyl units and 50 mole % ε-caproil units. Within the matrix, particles of propolis are uniformly dispersed in an amount of 13 % by weight. The thickness of the non-woven fabric is 400 µm, and the average diameter of the composite fibers is 1000 nm.

### Example 12

For the fabrication of non-woven fabric described in Example 11, 10 g of poly(L-lactide-co-ε-caprolactone) containing 50 mole % lactydyl units and 50 mole % ε-caproil units, were dissolved in 56 ml of 1,1,1,3,3,3-hexafluoro-2-propanol at room temperature to obtain a solution of 10 % by weight, and then 1.5 g of propolis was added and stirred on a magnetic stirrer at room temperature for 24 hours until it was completely dissolved. The solution obtained was introduced into a dispenser with a pump terminated with a nozzle, placed on a movable arm and moving along a cylindrical collector. The distance of the nozzle from the collector was 17 cm, and the voltage applied to the nozzle 25 kV. The cylinder with a diameter of 27 mm constituting the collector rotated at 800 rpm and was connected to a power supply with a negative potential of -5 kV. Composite fibers with an average diameter of 1000 nm were synthesized at a solution feed rate of 2.5 ml/h and deposited randomly on the collector surface for 16 h until a 400 µm thick non-woven fabric was obtained. The total volume of the solution used was 40 ml. The non-woven fabric was dried in a vacuum dryer at 10 mbar at 25°C for 10 days and then sterilized by electron beam at a dose of 10 kGy.

### Example 13

The non-woven fabric consists of randomly oriented composite fibers, composed in 80 % by weight of a biodegradable aliphatic copolymer, poly(glycolide-*co*-ε-caprolactone) containing 20 mole % glycolidyl units and 80 mole % ε-caproil units. Within the matrix, the propolis particles are uniformly dispersed in an amount of 20 % by weight. The thickness of the non-woven fabric is 340 µm, and the average diameter of the composite fibers 1000 nm.

### Example 14

In order to obtain the non-woven fabric described in Example 13, 8 g of poly(glycolide-co-ε-caprolactone) containing 20 mole % glycolidyl units and 80 mole % ε-caproil units was dissolved in 37 ml of 1,1,1,3,3,3-hexafluoro-2-propanol at room temperature to give a 12 % by weight solution, followed by the addition of 2 g propolis and stirred on a magnetic stirrer at room temperature for 24 hours until complete dissolution. The solution obtained was introduced into a dispenser with a pump terminated with a nozzle, placed on a movable arm and moving along a cylindrical collector. The distance of the nozzle from the collector was 17 cm, and the voltage applied to the nozzle 25 kV. The cylinder with a diameter of 27 mm constituting the collector rotated at 800 rpm and was connected to a power supply with a negative potential of -5 kV. Composite fibers with an average diameter of 1000 nm were synthesized at a solution feed rate of 2 ml/h and deposited randomly on the collector surface for 15 hours until a 340 µm thick non-woven fabric layer was obtained. The total volume of the solution used was 30 ml. The non-woven fabric was dried in a vacuum dryer at 10 mbar at 25°C for 10 days and then sterilized by electron beam at a dose of 10 kGy.

### Example 15

The non-woven fabric consists of randomly arranged composite fibers, composed in 50 % by weight of an aliphatic biodegradable copolymer, poly(lactide-co-trimethylene carbonate), with a 55 mole % of lactydyl units content and 45 mole % of trimethylene carbonate units content. Within the matrix, particles of propolis are uniformly dispersed in an amount of 50 % by weight. The thickness of the non-woven fabric is 300 µm, and the average diameter of the composite fibers is 1000 nm.

### Example 16

In order to obtain the non-woven fabric described in Example 15, 7 g of poly(lactide-co-trimethylene carbonate) with a lactydyl content of 55 mole % and carbonate units of 45 mole %, was dissolved in 35 ml of 1,1,1,3,3,3-hexafluoro-2-propanol at room temperature to give a 11 % by weight solution, followed by the addition of 7 g propolis and the mixture was stirred on a magnetic stirrer at room temperature for 24 hours until complete dissolution. The solution obtained was introduced into a dispenser with a pump terminated with a nozzle, placed on a movable arm and moving along a cylindrical collector. The distance of the nozzle from the collector was 17 cm, and the voltage applied to the nozzle 25 kV. The cylinder with a diameter of 27 mm constituting the collector rotated at 800 rpm and was connected to a power supply with a negative potential of -5 kV. Composite fibers with an average diameter of 1000 nm were synthesized at a solution delivery rate of 2 ml/h and deposited randomly on the collector surface for 14 h until a 300 µm thick non-woven fabric layer was obtained. The total volume of the solution used was 28 ml. The non-woven fabric was dried in a vacuum dryer at 10 mbar at 25°C for 10 days and then sterilized electron by beam at a dose of 10 kGy.

### Example 17

The non-woven fabric consists of randomly arranged composite fibers, composed in 90 % by weight of an aliphatic biodegradable copolymer, poly(glycolide-co-trimethylene carbonate), with a glycolidyl units content of 5 mole % and a carbonate units content of 95 mole %. Within the matrix, particles of propolis are uniformly dispersed in an amount of 10 % by weight. The thickness of the non-woven fabric is 320 µm, and the average diameter of the composite fibers is 1000 nm.

### Example 18

In order to obtain the non-woven fabric described in Example 17, 5 g of poly(glycolide-co-trimethylene carbonate), with a glycolidyl units content of 5 mole % and carbonate units content of 95 mole %, were dissolved in 36 ml 1,1,1,3,3,3-hexafluoro-2-propanol at room temperature to obtain a solution of 8 % by weight, and then 0.26 g of propolis was added and the mixture was stirred on a magnetic stirrer at room temperature for 24 hours until it was completely dissolved. The solution obtained was introduced into a dispenser with a pump terminated with a nozzle, placed on a movable arm and moving along a cylindrical collector. The distance of the nozzle from the collector was 17 cm, and the voltage applied to the nozzle 25 kV. The cylinder with a diameter of 27 mm constituting the collector rotated at 800 rpm and was connected to a power supply with a negative potential of -5 kV. Composite fibers with an average diameter of 1000 nm were synthesized at a solution feed rate of 2.5 ml/h and deposited randomly on the collector surface for 12 hours until a 300 µm thick non-woven fabric layer was obtained. The total volume of the solution used was 30 ml. The non-woven fabric was dried in a vacuum dryer at 10 mbar at 25°C for 10 days and then sterilized by electron beam at a dose of 10 kGy.

## Claims

1. A non-woven fabric dressing composed of randomly arranged fibers of a biodegradable polymer containing propolis, **characterized in that** the fibers are in the form of a composite with a biodegradable aliphatic copolymer matrix, constituting 25 to 99% by weight of the composite, and selected from the group consisting of:
(a) poly(lactide-co-glycolide) with a lactydyl content of 50 to 95 mole %, glycolidyl content of 5 to 50 mole %,
(b) poly(lactide-co-ε-caprolactone) with a lactydyl content of 5 to 95 mole %, ε-caproil content of 5 and 95 mole %.
(c) poly(glycolide-co-ε-caprolactone) with a glycolidyl content of 10 to 50 mole %, ε-caproil content of 50 and 90 mole %,
(d) poly(lactide-co-glycolide-co-ε-caprolactone) with a lactydyl content of 15 to 85 mole %, glycolidyl content of 5 to 50 mole %, ε-caproil content from 5 to 30 mole %,
(e) poly(lactide-co-trimethylene carbonate) with a lactydyl content of 5 to 95 mole %, trimethylene carbonate content from 5 to 95 mole %,
(f) poly(glycolide-co-trimethylene carbonate) with a glycolidyl content of 5 to 30 mole %, trimethylene carbonate content of 70 to 95 mole %,
(g) poly(lactide-co-glycolide-co-trimethylene carbonate) with a lactydyl content of 15 to 85 mole %, glycolidyl content of 5 to 50 mole %, trimethylene carbonate content from 5 to 50 mole %.
with the particles of propolis uniformly dispersed in the biodegradable copolymer matrix in an amount of 1 to 75 % by weight, wherein non-woven fabric thickness ranges from 20 to 2000 µm, and the average diameter of the composite fibers ranging from 250 to 5000 nm.

2. A non-woven fabric according to claim 1, wherein the propolis is dispersed in a matrix of a biodegradable polymer in an amount of 5 to 20 % by weight.

3. A non-woven fabric according to claim 1, wherein thickness of the non-woven fabric is from 200 to 500 µm.

4. A non-woven fabric according to claim 1, wherein mean diameter of the composite fibers forming the non-woven fabric ranges from 500 to 1000 nm.

5. A method of producing a non-woven dressing fabric, composed of randomly arranged fibers of a biodegradable polymer, containing propolis, made by electrospinning method, which consists in formation in the electric field of the fibers from a polymer solution which is placed in a nozzle-equipped dispenser connected with an infusion pump, and then in deposition of the fibers randomly on the collector surface, **characterized in that** the fibers are in the form of a composite with a biodegradable aliphatic copolymer matrix, constituting 25 to 99% by weight of the composite, and selected from the group consisting of:
(a) poly(lactide-co-glycolide) with a lactydyl content of 50 to 95 mole %, glycolidyl content of 5 to 50 mole %,
(b) poly(lactide-co-ε-caprolactone) with a lactydyl content of 5 to 95 mole %, ε-caproil content of 5 and 95 mole %.
(c) poly(glycolide-co-ε-caprolactone) with a glycolidyl content of 10 to 50 mole %, ε-caproil content of 50 and 90 mole %,
(d) poly(lactide-co-glycolide-co-ε-caprolactone) with a lactydyl content of 15 to 85 mole %, glycolidyl content of 5 to 50 mole %, ε-caproil content from 5 to 30 mole %,
(e) poly(lactide-co-trimethylene carbonate) with a lactydyl content of 5 to 95 mole %, trimethylene carbonate content from 5 to 95 mole %,
(f) poly(glycolide-co-trimethylene carbonate) with a glycolidyl content of 5 to 30 mole %, trimethylene carbonate content of 70 to 95 mole %,
(g) poly(lactide-co-glycolide-co-trimethylene carbonate) with a lactydyl content of 15 to 85 mole %, glycolidyl content of 5 to 50 mole %, trimethylene carbonate content from 5 to 50 mole %
wherein the aliphatic biodegradable copolymer is dissolved in a fluorinated alcohol, preferably 1,1,1,3,3,3-hexafluoro-2-propanol, at room temperature, obtaining the solution of a concentration from 1 to 20 % by weight, followed by addition of propolis in amount from 1 to 75 % by weight to above solution and mixing for up to 24 hours until it is completely dissolved, and the obtained base solution is then introduced into a dispenser with infusion pump terminated with a spinning nozzle placed on a movable arm and moving along the collector, preferably cylindrical and rotating, and the nozzle distance from the collector is from 5 cm to 30 cm, and the potential difference between the nozzle and the collector is from 5 to 50 kV, and composite fibers with a mean diameter of 250 to 5000 nm are then formed, at a feed rate of the base solution from 0.1 to 15 ml/h, preferably from 1 to 2.5 ml/h, deposited on the surface of the collector until a non-woven fabric is obtained with a thickness in the range from 20 to 2000 µm, and when the electrospinning process is finished, the non-woven fabric is dried in a vacuum dryer for up to 10 days and then subjected to e-beam or gamma irradiation sterilization.

6. A method according to claim 5, wherein the propolis is added in an amount ranging from 5 to 20 % by weight.

7. A method according to claim 5, wherein the composite fibers are formed with an average diameter of 500 to 1000 nm.

8. A method according to claim 5, wherein the fibers are arranged to obtain a non-woven fabric with a thickness in the range from 200 to 500 µm.

9. A method according to claim 5, wherein the biodegradable copolymer solution is prepared in the concentration ranging from 6 to 10 % by weight.
